# EUROPEAN PATENT SPECIFICATION

(11) **EP 0 474 270 B1**
(45) Date of publication and mention of the grant of the patent: **20.11.1996**
(21) Application number: 91201817.3
(22) Date of filing: 11.07.1991
(51) Int. Cl.: A61K 7/48, A61K 7/00, A61K 7/025, A61K 7/027

(54) **Cosmetic composition**
Kosmetische Zusammensetzung
Composition cosmétique

(30) Priority: 25.07.1990 US 558140
(43) Date of publication of application: 11.03.1992
(73) Proprietor: UNILEVER N.V., 3013 AL Rotterdam (NL); UNILEVER PLC, London EC4P 4BQ (GB)
(72) Inventor: Dunphy, Patrick Joseph, Sharnbrook, Bedford MK44 1LQ (GB); Meyers, Alan Joel, Edgewater, NJ 07020 (US); Rigg, Richard Tyson, Edgewater, NJ 07020 (US)
(74) Representative: Mulder, Cornelis Willem Reinier, Dr.

(56) References cited:
- EP-A- 0 062 896
- EP-A- 0 084 341
- EP-A- 0 349 150
- WO-A-81/02673
- US-A- 4 115 313
- PATENT ABSTRACTS OF JAPAN vol. 11, no. 38 (C-401)(2485) 4 February 1987 & JP-A- 61 204 109 ( EISAI CO LTD ) 10 September 1986
- WORLD PATENTS INDEX LATEST Week 8623, Derwent Publications Ltd., London, GB; AN 86-147740 & JP-A- 61 023 110 (KONUKI S) 26 April 1986
- PATENT ABSTRACTS OF JAPAN vol. 1, no. 120 (C-343)6 May 1986 & JP-A- 60 246 305 ( NISSHIN SEIYU KK ) 6 December 1985

## Description

### Field of the Invention

The invention relates to a cosmetic emulsion composition, particularly to an emulsion which is adapted to be applied to human skin. The cosmetic emulsion is particularly useful for depositing thereon a protective and/or therapeutic and/or coloured film, and can be used in a variety of product forms, particularly cosmetic product forms, including lipstick, blusher, eyeliner, mascara, and lip balms or lip glosses, but is especially suitable for lip products. The invention is accordingly particularly concerned with an improved emulsion lipstick having an enhanced in use feel, moisturisation, protection, and reparative properties.

### Background and Prior Art

In conventional cosmetic product manufacture in general, but especially in the manufacture of lipsticks, fats and/or oils, together with pigments and/or lakes, and other non-aqueous ingredients, are usually added to a wax base which is melted to enable the ingredients to be thoroughly mixed and is then cast into moulds which, after cooling, provide the shaped product. In for example commercially available lipsticks, water is not usually incorporated into the lipstick formulation, and therefore, the lipstick when applied to the lips does not necessarily possess the smooth, soft attributes associated with other skin treatment products, such as skin creams, particularly those intended for moisturising the lips, or other parts of the skin.

It is accordingly desirable to provide for some users a lipstick that has more of the moisturising attributes of skin products, such as those referred to above, than conventional water-free lipsticks.

It has been proposed in JP-A-61/83110 (Konuki) to incorporate water into a lipstick to achieve in use a fat/oil coating containing water on the lip surface, to provide, so it is stated, healthy, beautiful lips. Moreover, the product is said to have a soft and good feeling in use. To achieve this, dispersing agents such as cholesterol, phytosterol, phospholipids and/or saponins are employed. Also, anionic surfactants, cationic surfactants, nonionic surfactants or amphoteric surfactants may be used so that water or a solution of water-soluble substances in water can be more homogenously and stably dispersed in this type of product.

Our attempts to repeat the teaching of JP-A-61/83110 have resulted in lipsticks which, although possessing some moisturising capacity in view of the small amount of water present, still do not meet the requirements of the more discerning user, in that the products are too soft and/or do not glide easily over the lip surface during application and/or leave an imperfect film or undesirable after feel on the lip surface following application. Furthermore, it has been found that products according to the teaching of JP-A-61/83110 (see for example formulation 3) have been found to have undesirable storage characteristics. In particular, in accelerated storage tests conducted at 50°C (to simulate the effects of longer term storage tests conducted at lower temperatures) it has been found that formulations according to JP-A-61/83110 have a tendency to display "sweating" i.e. oil in the formulations leaches out to the surface of the lipstick within a very short time (i.e. 1 day or less). It is thought that this could well be due to the fact that a large proportion (around 73%) of the ingredients of the formulations of JP-A-61/83110 are liquid at room temperature.

It is accordingly with the avoidance of these disadvantages, particularly with improving moisturisation of the lips and delivery thereto of skin care active ingredients that this invention is concerned.

### Summary of the Invention

We have now discovered that by employing a special mixture of emulsifiers, a superior water-in-oil lipstick can be obtained which possesses none of the negative attributes of the lip products made according to the teaching of JP-A-61/83110.

### Definition of the Invention

A water-in-oil cosmetic emulsion suitable for use as a stick formulation which comprises:
i) from 30 to 97% by weight of oil;
ii) from 1 to 25% by weight of wax;
iii) from 1 to 20% by weight of water;
iv) from 0.2 to 10 % by weight of a phospholipid; and
v) from 0.2 to 10% by weight of a glycerol derivative having a melting point of from -20 to 80°C.

### Disclosure of the Invention

The invention is concerned with an improved cosmetic emulsion, particularly a lipstick, which comprises a very special emulsifier system that imparts to the emulsion a consumer-perceivable improved in-use benefits.

The function of the special emulsifier system is not only to provide these in-use benefits, but also to aid the delivery to the skin of the product, particularly the lips, of skin care active ingredients, as hereinafter disclosed, which can function to provide moisturisation, emollience and other improvements.

According to a particularly preferred embodiment of the invention, formulations according to the invention should comprise no more than 68% by weight of the formulation of components which are liquid at room temperature, preferably not more than 65% by weight of components which are liquid at room temperature, most preferably no more than 60% by weight of components which are liquid at room temperature.

### The emulsifier system

The emulsifier system comprises, as a first emulsifier, a phospholipid, which contributes to the stability and pleasing appearance of the emulsion. Examples of three classes of phospholipids are phosphoglycerides, lysophosphoglycerides and the important but smaller group of sphingomyelins. It is also possible to employ a mixture of two or more of these phospholipids, which together would then comprise the first emulsifier.

Examples of phosphoglycerides include:
- phosphatidyl choline,
- phosphatidyl ethanolamine,
- phosphatidyl serine,
- phosphatidyl inositol,
- diphosphatidyl glycerol
- ammonium phosphatides (e.g. Chocothin YN (Trade Mark) of general structure 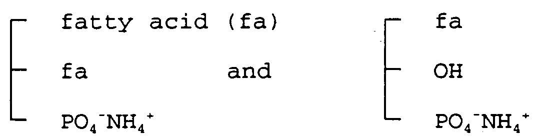
- lyso and phospholipids from egg containing more saturate fatty acids (e.g. Ovathins 120-200 (Trade Mark)) or synthetic analogues with saturated fatty acids from C10 and C20.
- mono and/or dialkyl and mono and di-alkenyl phosphates or mixtures of the same e.g. dicetyl and dioleyl phosphates and oleth-n-phosphates (where n=3 to 50).
- sphingomyelins consisting of single fatty acyl chain conjugated via an amide linkage to the nitrogen of sphingosine which is again linked to phosphoryl choline or other bases.
- ceramides in which the phosphoryl base head group of sphingomyelins is absent.
- cerebrosides and gangliosides which contain polysaccharide head groups in place of the phosphoryl base groups of sphingomyelins.
- plasmogens in which the 1' position is occupied by an ether linked alk-l-enyl chain.
- cardiolipin in which two molecules of diacyl glycerol phosphatide are linked by a molecule of glycerol and mixtures thereof.

The most preferred phosphoglyceride is that known as lecithin, particularly soyabean lecithin, which comprises a mixture of some of the above examples of specific phosphoglycerides.

Examples of lysophosphoglycerides includes:
lysophosphatidyl choline,
lysophosphatidyl ethanolamine,
lysophosphatidyl serine,
lysophosphatidyl inositol, and
mixtures thereof

The amount of phospholipid normally present in the cosmetic emulsion of the invention is from 0.2 to 10%, preferably from 0.5 to 2% by weight of the cosmetic emulsion.

The emulsifier system also comprises, in addition to the phospholipid, a second emulsifier having a melting point of from -20°C to 80°C, preferably -10 to 60°C, more preferably from -5 to 50°C. It is also possible to employ a mixture of two or more of these second emulsifiers, which together would then comprise the second emulsifier.

The second emulsifier comprises derivatives of glycerol, all of which have a melting point in the range -20°C to 80°C, including:
i) monoacyl (mainly 1-substituted) glycerol, such as glyceryl monoalkanoates, in which the alkanoate group has from 2 to 20 carbon atoms, with or without one or more hydroxyl groups, and is branched or unbranched, specific examples of which are:
   glyceryl caprylate
   glyceryl caprate
   glyceryl laurate
   glyceryl myristate
   glyceryl palmitate
   glyceryl stearate
   glyceryl isostearate
   or glyceryl monoalkenoates, in which the alkenoate group has from 3 to 20 carbon atoms, with or without one or more hydroxyl groups, and is branched or unbranched, and has from 1 to 4 double bonds, specific examples of which are:
   glyceryl oleate
   glyceryl linoleate
   glyceryl ricinoleate.
ii) diacyl (1,2- or 1,3- di substituted) glycerol, such as glyceryl dialkanoates, in which the alkanoate groups each have from 2 to 20 carbon atoms, with or without one or more hydroxyl groups, and each are branched or unbranched, specific examples of which are:
   glyceryl diacetate,
   glyceryl dibutanoate,
   glyceryl dilaurate,
   glyceryl dicaprate.
   or glyceryl dialkenoates, in which the alkenoate groups each have from 3 to 20 carbon atoms, with or without one or more hydroxyl groups, and each are branched or unbranched, and have from 1 to 4 double bonds, specific examples of which are:
   glyceryl dioleate
   glyceryl dilinoleate.
iii) polyglyceryl esters, in which up to 5 glycerol residues are linearly linked with one to two acyl groups each having from 2 to 20 carbon atoms, with or without one or more hydroxyl groups, are each branched or unbranched, saturated or unsaturated, specific examples of which are polyglyceryl ricinoleate and polyglycerol-3-monooleate.

other preferred glycerol derivatives include:
- glycerol ether derivatives of the above mono and diacyl types, for example CH₂OH. CHOH. CH₂OX. Examples include where X = CH₃(CH₂)₁₇ (batyl alcohol), and where X = CH₃(CH₂)₁₅CH=CH (selachyl alcohol).
- mixed acyl, ether derived glycerols, including the neutral plasmogens which are structurally 1'-alk-1-enyl, 2'3' diacyl glycerols.
- polyglycerol esters, including polyglyceryl polyricinoleate and fatty esters of blown soya bean oil (Homodan PT-trademark).
- polyhydethers and esters of saturated and unsaturated alcohols and fatty acids respectively, particularly with ethylene, propylene, butylene glycols and polyethylene glycols.
- betaines of general form R-N(Me)₂CH₂COOH.

Particularly preferred examples of the second emulsifiers are:

| | melting point |
|---|---|
| glyceryl caprate: | 40°C, |
| glyceryl laurate: | 51°C, |
| glyceryl myristate: | 61°C, |
| glyceryl palmitate: | 69°C, |
| glyceryl stearate: | 75°C, |
| glyceryl oleate: | 35°C, |
| glyceryl linoleate: | 12°C, |
| glyceryl isostearate: | 5°C, |
| glyceryl dilinoleate: | -3°C, |
| glyceryl dicaprate: | 44°C. |

The melting points quoted above are applicable to the most stable, highest melting forms in each case.

The second emulsifier can comprise one or more pure glycerides, but it is usually more convenient to employ glycerides derived from natural oils such as vegetable or seed oils, examples of which are sunflower seed oil, soyabean oil, palm kernel oil and palm oil. Particularly preferred are monoglycerides of sunflower seed oil and of palm oil, and mono-and di-glycerides of soyabean oil.

Further examples of the second emulsifier include esters of fatty alcohols with a hydroxy acid, such as cetyl recinoleate, glyceryl citrate, cetyl citrate and cetyl lactate (having a melting point of 24°C).

The amount of the second emulsifier normally present in the cosmetic emulsion of the invention is from 0.2 to 10%, more preferably 0.5 to 10%, most preferably from 0.5 to 5% by weight of the cosmetic emulsion.

It is apparent that cosmetic emulsions containing less than 0.2% by weight of either the first or the second emulsifier can be unstable and lack the desirable pleasing appearance attributable to the presence of a higher level of either of these emulsifiers.

It has been found that optimal qualities in a formulation for application to the lips are achieved when the formulation contains at least about 0.5% by weight of each emulsifier, or more than about 1% in total of the combined emulsifiers.

The cosmetic emulsion contains less than about 10% in total of either emulsifier.

In many of the preferred formulations according to the invention, the HLB value of the emulsifier system is between 4 and 8, more preferably 4 and 6.

### The oil

The cosmetic emulsion of the invention also comprises an oily ingredient, hereinafter referred to as an "oil" which together with water and the emulsifier system, forms a water-in-oil or oil-in-water emulsion.

A chosen oil will normally be liquid at room temperature (i.e. 20°C), and can comprise a single oil or a mixture of two or more oils. Example of suitable oils include:
caprylic triglycerides
capric triglycerides
isostearic triglycerides
adipic triglycerides
propylene glycol myristyl ether acetate
lanolin oil
polybutene
isopropyl palmitate
isopropyl myristate
diethyl sebacate
diisopropyl adipate
hexadecyl stearate
cetyl oleate
oleyl alcohol
hexadecyl alcohol
wheatgerm oil
hydrogenated vegetable oils
petrolatum
modified lanolins
branched-chain hydrocarbons, alcohols and esters
castor oil
corn oil
cotton seed oil
olive oil
palm kernal oil
rapeseed oil
safflower seed oil
jojoba oil
evening primrose oil
avocado oil
mineral oil
volatile and non-volatile silicone oils

The amount of the oil normally present in the cosmetic emulsion of the invention is from 30 to 97%, preferably 30 to 90% by weight of the cosmetic emulsion.

### Water

The cosmetic emulsion of the invention also comprises water which forms the basis of the aqueous phase of the emulsion and provides a solvent for any water-soluble ingredients present in the cosmetic emulsion.

The amount of water present in the cosmetic emulsion of the invention is from 1 to 20%, the preferred amount depending on the product form of the cosmetic emulsion.

### Wax

The cosmetic emulsion of the invention comprises one or more waxy ingredients, hereinafter referred to as a "wax".

Examples of waxes include:
candelilla wax
ozokerite wax
carnauba wax
beeswax
spermaceti
lanolin
cetyl alcohol
stearyl alcohol
high melting point parafinic hydrocarbons.

The amount of wax present in the cosmetic emulsion of the invention is from 1 to 25% and, preferably from 5 to 20% by weight of the cosmetic emulsion.

### Skin care active ingredients

The cosmetic emulsion of the invention can optionally also comprise skin care active ingredients which can improve the quality of skin, particularly dry or damaged skin especially the lips, or which can possess a therapeutic or pharmaceutical benefit. By way of example, the following skin care active ingredients can be employed, in the amounts stated, in lip treatment emulsions of the invention, especially in lipsticks.

| | % w/w |
|---|---|
| zinc oxide | 1 to 2 |
| **β**-glycyrrhetic acid | 0.1 to 1 |
| camomile oil | 0.1 to 1 |
| ginko biloba extract | 0.1 to 1 |
| pyroglutamic acid, salts or esters | 0.5 to 5 |
| sodium hyaluronate | 0.1 to 5 |

For the treatment of spots, pimples and acne comedones, additional therapeutically or pharmaceutically active ingredients can be employed. Examples are:

| | % w/w |
|---|---|
| 2-hydroxyoctanoic acid | 0.5 to 5 |
| sulphur | 1 to 10 |
| salicylic acid | 1 to 3 |
| carboxymethyl cysteine | 0.1 to 5 |

The amount of skin care active ingredients when present in the cosmetic emulsion of the invention will normally be from 0.0001 to 10% by weight of the cosmetic emulsion. In general terms it can be stated that the amount of any skin care active ingredient can be that which is conveniently employed in products intended for topical application to human skin.

### Other ingredients

The cosmetic emulsion of the invention can optionally also comprise other ingredients as conventionally employed in lipsticks or other lipcare products.

Examples of other ingredients include perfumes, antioxidants, colorants such as staining dyes and pigments, humectant, germicides, sunscreens, lipid materials, and vitamins.

Particularly preferred pigments, when present, include calcium, barium and aluminium lakes, iron oxides, titanium dioxide, and mica.

Particularly preferred humectant include glycerol, sorbitol and other polyols.

Particularly preferred germicides include Triclosan.

Particularly preferred sunscreens include octyl methoxycinnamate and butyl methoxydibenzoylmethane.

Particularly preferred lipid materials include ceramides and liposomes.

Particularly preferred vitamins include vitamin A, C, D and E.

### Product form

When used as a lip formulation (i.e. a pigmented lipstick formulation, or a lip balm or gloss) the cosmetic emulsion of the invention can take the form of a solid stick, which can be applied to the lips with a suitable applicator.

The emulsion is a water-in-oil emulsion, the amount of each phase present being governed by the form of the product itself.

A preferred embodiment of the invention is a water-in-oil emulsion lipstick comprising:
i from 30 to 97%, most preferably from 70 to 80%, by weight of oil
ii. from 1 to 25%, most preferably from 12 to 20%, by weight of wax
iii. from 1 to 20%, most preferably from 1 to 10%, by weight of water
iv. a sufficient amount of an emulsifier system comprising:
   a. from 0.2 to 10%, most preferably 0.2 to 4%, by weight of a phospholipid, and
   b. from 0.2 to 10%, most preferably from 0.2 to 4%, by weight of a glyceryl monoalkenoate, in which the alkenoate group has from 3 to 20 carbon atoms; and
v. a sufficient amount of pigment.

### Use of the Emulsion

The cosmetic emulsion of the invention is ideally suited for use as a stick formulation in treating the lips, especially for applying to the lips a permanent or semi-permanent colour, ideally with a gloss or lustre finish.

The emulsion can also be used in treating the lips with a skin care agent for protection against exposure to adverse weather, including the wind and the rain or exposure to excessive doses of sunlight.

The cosmetic emulsion in the form of a stick, can accordingly be applied to the lips in the traditional manner using a convenient holder or applicator to provide a decorative and/or protective film thereto.

The cosmetic emulsion according to the invention is also suitable to act as the base material for a variety of make up formulations, including mascara, eyeliner and blusher.

### Examples

The invention is further illustrated by the following examples.

### Example 1

A cosmetic emulsion lipstick in accordance with the invention contained the following ingredients:

| **Ingredient** | **% w/w** |
|---|---|
| | |

| A. **Oily Phase** | |
|---|---|
| **Oil** | |
| caprylic/capric triglyceride | 5.8 |
| propylene glycol myristyl ether acetate | 6.0 |
| lanolin oil | 2.5 |
| polybutene | 0.8 |
| caprylic/capric/isostearic/adipic triglyceride | 7.0 |
| isopropyl palmitate | 11.6 |

| **Wax** | |
|---|---|
| candelilla wax | 6.6 |
| ozokerite wax | 2.5 |
| carnauba wax | 0.4 |
| beeswax | 4.1 |
| lanolin | 7.0 |

| **Emulsifier system** | |
|---|---|
| phospholipid, namely soyabean lecithin | 1.0 |
| glyceride, namely Hymono 7804* | 3.5 |

| B. **Aqueous phase** | |
|---|---|
| glycerol | 5.0 |
| water | 5.0 |

| C. **Pigments dispersed in castor oil** | |
|---|---|
| titanium dioxide | 4.7 |
| colourants | 7.0 |
| castor oil | 19.5 |

| | |
|---|---|
| * Hymono 7804 is distilled monoglyceride (>90%) of vegetable oil origin. | |

The lipstick having the above formulation was manufactured as follows:
1. The combined wax, oil and emulsifier which comprise the oily phase (A) were heated and stirred in a sealed vessel to a temperature of 90°C, until the mass has melted.
2. The melt so obtained was then cooled to 75-80°C and stirred under vacuum to remove air.
3. The aqueous phase comprising water and water-soluble ingredients (B) was heated to a temperature of 75-80°C and then slowly added, with mixing, to the molten oily phase (A) and the mixture homogenised for from 5 to 10 minutes.
4. Pigments and fragrance dispersed in castor oil (C) were finally added and the water-in-oil emulsion so formed was further deaerated prior to cooling.
5. The deaerated emulsion was finally poured into moulds and cooled to form lipsticks.

### Examples 2 to 12

Examples 2 to 12 illustrate further cosmetic emulsion lipsticks according to the invention. In each example the emulsifier was varied, while the remaining ingredients remained unchanged.

The phospholipid emulsifier employed in each case was as follows:

| **Example No.** | **Phospholipid** | **% w/w** |
|---|---|---|
| 2 | Soya lecithin | 1 |
| 3 | Soya lecithin | 1 |
| 4 | Soya lecithin | 1 |
| 5 | Soya lecithin | 1 |
| 6 | Soya lecithin | 1 |
| 7 | Soya lecithin | 0.5 |
| 8 | Soya lecithin | 2 |
| 9 | Soya lecithin | 1 |
| 10 | Soya lecithin | 1 |
| 11 | Lyso-soya lecithin | 1 |
| 12 | Phosphatidyl choline-enriched soya lecithin | 1 |

The glyceride emulsifier employed in each case was as follows:

| **Example No.** | **Glyceride** | **% w/w** |
|---|---|---|
| 2 | Sunflower oil monoglyceride | 3.5 |
| 3 | Glyceryl isostearate | 3.5 |
| 4 | Glyceryl caprate | 3.5 |
| 5 | Sunflower oil monoglyceride | 2.5 |
| 6 | Sunflower oil monoglyceride | 5 |
| 7 | Sunflower oil monoglyceride | 3.5 |
| 8 | Sunflower oil monoglyceride | 3.5 |
| 9 | Soyabean oil mono/diglyceride | 3.5 |
| 10 | Polyglyceryl ricinoleate | 3.5 |
| 11 | Sunflower oil monoglyceride | 3.5 |
| 12 | Sunflower oil monoglyceride | 3.5 |

### Example 13

A cosmetic emulsion lip gloss in accordance with the invention contained the following ingredients:

| A) **Oily phase** | **% w//w** |
|---|---|
| **Oil** | |
| polybutene | 34 |
| lanolin oil | 20 |
| preservative | 1 |
| PPG-5-lanolin wax | 13 |

| **Wax** | |
|---|---|
| lanolin wax | 13 |

| **Emulsifier system** | |
|---|---|
| soya lecithin | 1 |
| sunflower oil monoglycerides | 3.5 |

| B) **Aqueous phase** | |
|---|---|
| glycerol | 5 |
| water | 5 |

| C) **Pigments dispersed in lanolin oil (as above)** | |
|---|---|
| titanium dioxide | 3 |
| colourants | 1.5 |

### Examples 14 & 15

Examples 14 & 15 illustrate further cosmetic emulsion lip gloss products according to the invention. In each case, the formulation was similar to that of Example 13 except that the aqueous phase or the emulsifier system was varied as follows:

In Example 14, the aqueous phase formed 20% w/w of the formulation (1:1 glycerol:water) with all other ingredients, except the emulsifier system being reduced proportionately.

In example 15, the emulsifier was 1.0% soya lecithin and 3.5% distilled monoglyceride (>90%) based on palm oil.

### Example 16

This example illustrates a further cosmetic emulsion lipstick according to the invention. The formulation and processing were identical to Example 1 except that the aqueous phase was varied as follows:-

In Example 16, the aqueous phase included 2.5% by weight, in terms of the finished product, of a lecithin liposome ingredient based on dispersed and included sodium hyaluronate. The liposome ingredient contained 0.1% by weight sodium hyaluronate and 10% by weight lecithin and had a maximum particle size of 100nm.

### Examples 17-19

Cosmetic emulsion lipsticks/lipbalm formulations in accordance with the invention contained the following ingredients, and were prepared according to the method of example 1.

### Example 17

| **Ingredient** | **Parts by weight of the composition** |
|---|---|
| Squalene | 3.2 |
| Vegetable fatty acids | 12.4 |
| Assorted oils | 47.1 |
| Glycerol tripalmitate | 7.0 |
| Cholesterol/esters | 2.0 |
| Mica | 0.5 |
| Sunflower oil monoglycerides | 3.0 |
| Soya lecithin | 1.0 |
| Antioxidants | 0.6 |
| Caprylic/capric/isostearic/Adipic triglycerides | 9.0 |
| H₂O | 9.0 |
| Glycerol | 5.0 |

### Example 18 - Vegetable oil based lipstick formulation

| **Ingredient** | **Parts by weight of the composition** |
|---|---|
| Tristearin | 6.4 |
| Tripalmitin | 3.3 |
| Beeswax | 3.9 |
| Trilarin | 4.9 |
| Caprylic/capric triglyceride | 5.9 |
| Caprylic/capric/isostearic/Adipic triglycerides | 8.8 |
| Sunflower oil | 19.6 |
| Vit. E linoleate | 0.5 |
| BHA | 0.1 |
| Soya lecithin | 1.0 |
| Sunflower oil monoglycerides | 5.1 |
| Mica UF | 1.0 |
| Castor oil/natural pigment | 25.8 |
| Water | 8.8 |
| Glycerol | 4.9 |

### Example 19 - Lipbalm formulation

| A. **Oily Phase** | **Parts by weight of the composition** |
|---|---|
| **Oil** | |
| Caprylic/capric triglyceride | 5.8 |
| Propylene glycol myristyl ether acetate | 6.0 |
| Lanolin oil | 2.5 |
| Caprylic/capric/isostearic/adipic triglyceride | 9.6 |
| Isopropyl palmitate | 11.6 |

| **Wax** | |
|---|---|
| Candelilla Wax | 11.4 |
| Ozokerite Wax | 5.2 |
| Carnauba Wax | 0.8 |
| Beeswax | 7.1 |
| Lanolin | 5.0 |

| **Emulsifier system** | |
|---|---|
| Soya lecithin | 1.0 |
| Sunflower oil monoglyceride | 3.5 |

| B. **Aqueous Phase** | |
|---|---|
| Glycerol | 5.0 |
| Water | 3.2 |

| C. **Pigments dispersed in Castor Oil** | |
|---|---|
| Colourant | 0.1 |
| Castor oil | 21.6 |

| D. **Moisturiser** | |
|---|---|
| Sodium pyrrolidone carboxylic acid | 0.6 |

### Example 20

| Crayon Lipstick | % w/w |
|---|---|
| Beeswax | 2.0 |
| Candelilla Wax | 5.0 |
| Ozokenite | 8.0 |
| Carnauba Wax | 2.5 |
| Lanolin | 5.0 |
| Isopropyl myristate | 10.0 |
| Oleyl alcohol | 16.0 |
| Mineral oil 65/75 | 5.0 |
| Preservative | 0.1 |
| Antioxidant | 0.1 |
| Barium Lake | 5.0 |
| Castor oil | 20.8 |
| Perfume | 1.0 |
| Sunflower Oil Monoglyceride | 3.5 |
| Soya lecithin | 1.0 |
| Water | 3.0 |
| Glycerol | 5.0 |
| Titanium Dioxide/Mica | 5.0 |

### Example 21

### Lip Balm Formulations

| | % w/w |
|---|---|
| Hydrocarbon Wax | 15.0 |
| Petroleum jelly | 69.6 |
| Camphor | 0.6 |
| Perfume | 0.3 |
| Soya Lecithin | 1.0 |
| Sunflower Oil Monoglyceride | 3.5 |
| Glycerol | 5.0 |
| Sodium PCA | 1.0 |
| Water | 4.0 |

## Claims

1. A water-in-oil cosmetic emulsion suitable for use as a stick formulation which comprises:
i) from 30 to 97% by weight of oil;
ii) from 1 to 25% by weight of wax;
iii) from 1 to 20% by weight of water;
iv) from 0.2 to 10 % by weight of a phospholipid; and
v) from 0.2 to 10% by weight of a glycerol derivative having a melting point of from -20 to 80°C.

2. A cosmetic emulsion according to claim 1 wherein the glycerol derivative has a melting point of from -15 to 60°C.

3. A cosmetic emulsion according to claim 1 or 2 wherein the phospholipid is selected from phosphoglycerides, lysophosphoglycerides, sphingomyelins and mixtures thereof.

4. A cosmetic emulsion according to claim 3 wherein the phosphoglyceride is selected from phosphatidyl choline, phosphatidyl ethanolamine, phosphatidyl serine, phosphatidyl inositol, diphosphatidyl glycerol and mixtures thereof.

5. A cosmetic emulsion according to claim 3 wherein the phosphoglyceride is a lecithin.

6. A cosmetic emulsion according to claim 3 wherein the lysophosphoglyceride is selected from lysophosphatidyl choline, lysophosphatidyl ethanolamide, lysophosphatidyl serine, lysophosphatidyl inositol and mixtures thereof.

7. A cosmetic emulsion according to any one of the preceding claims wherein the glycerol derivative is selected from glyceryl monoalkanoates in which the alkanoate group has from 2 to 20 carbon atoms, glyceryl monoalkenoates in which the alkenoate group has from 3 to 20 carbon atoms, and mixtures thereof.

8. A cosmetic emulsion according to any one of the preceding claims 1 to 6 wherein the glycerol derivative is a diglyceride selected from glyceryl dialkanoates in which the alkanoate groups each have from 2 to 20 carbon atoms, glyceryl dialkenoates in which the alkenoate groups each have from 3 to 20 carbon atoms, and mixtures thereof.

9. A cosmetic emulsion according to any of the preceding claims which is in the form of a water-in-oil emulsion lipstick.

10. A cosmetic emulsion according to claim 9 which further comprises a sufficient amount of pigment.

11. A cosmetic emulsion according to any one of the preceding claims wherein less than 65 % by weight of the components of the cosmetic emulsion are liquid at room temperature.

## Patentansprüche

1. Kosmetische Wasser-in-Öl-Emulsion, geeignet zur Verwendung als Stiftformulierung, umfassend:
i) 30 bis 97 Gew.-% Öl;
ii) 1 bis 25 Gew.-% Wachs;
iii) 1 bis 20 Gew.-% Wasser;
iv) 0,2 bis 10 Gew.-% eines Phospholipids; und
v) 0,2 bis 10 Gew.-% eines Glycerinderivats mit einem Schmelzpunkt von -20 bis 80°C.

2. Kosmetische Emulsion nach Anspruch 1, dadurch gekennzeichnet, daß das Glycerinderivat einen Schmelzpunkt von -15 bis 60°C hat.

3. Kosmetische Emulsion nach Anspruch 1 oder 2, dadurch gekennzeichnet, daß das Phospholipid ausgewählt ist aus Phosphoglyceriden, Lysophosphoglyceriden, Sphingomyelinen und deren Gemischen.

4. Kosmetische Emulsion nach Anspruch 3, dadurch gekennzeichnet, daß das Phosphoglycerid ausgewählt ist aus Phosphatidylcholin, Phosphatidylethanolamin, Phosphatidylserin, Phosphatidylinositol, Diphosphatidylglycerin und deren Gemischen.

5. Kosmetische Emulsion nach Anspruch 3, dadurch gekennzeichnet, daß das Phosphoglycerid ein Lecithin ist.

6. Kosmetische Emulsion nach Anspruch 3, dadurch gekennzeichnet, daß das Lysophosphoglycerid ausgewählt ist aus Lysophosphatidylcholin, Lysophosphatidylethanolamid, Lysophosphatidylserin, Lysophosphatidylinositol und deren Gemischen.

7. Kosmetische Emulsion nach einem der vorhergehenden Ansprüche, dadurch gekennzeichnet, daß das Glycerinderivat ausgewählt ist aus Glycerylmonoalkanoaten, in denen die Alkanoatgruppe 2 bis 20 Kohlenstoffatome hat, Glycerylmonoalkenoaten, in denen die Alkenoatgruppe 3 bis 20 Kohlenstoffatome hat, und deren Gemischen.

8. Kosmetische Emulsion nach einem der vorhergehenden Ansprüche 1 bis 6, dadurch gekennzeichnet, daß das Glycerinderivat ein Diglycerid ist, ausgewählt aus Glyceryldialkanoaten, in denen die Alkanoatgruppen jeweils 2 bis 20 Kohlenstoffatome enthalten, Glyceryldialkenoaten, in denen die Alkenoatgruppen jeweils 3 bis 20 Kohlenstoffatome enthalten, und deren Gemischen.

9. Kosmetische Emulsion nach einem der vorhergehenden Ansprüche, dadurch gekennzeichnet, daß sie in der Form eines Wasser-in-Öl-Emulsions-Lippenstiftes vorliegt.

10. Kosmetische Emulsion nach Anspruch 9, dadurch gekennzeichnet, daß sie ferner eine hinreichende Menge an Pigmenten enthält.

11. Kosmetische Emulsion nach einem der vorhergehenden Ansprüche, dadurch gekennzeichnet, daß weniger als 65 Gew.-% der Komponenten der kosmetischen Emulsion bei Raumtemperatur flüssig sind.

## Revendications

1. Emulsion cosmétique eau dans l'huile convenant à une utilisation en formulation en stick, qui comprend:
i) de 30 à 97% en poids d'huile;
ii) de 1 à 25% en poids de cire;
iii) de 1 à 20% en poids d'eau;
iv) de 0,2 à 10% en poids d'un phospholipide; et
v) de 0,2 à 10% en poids d'un dérivé de glycérol ayant un point de fusion de -20 à 80°C.

2. Emulsion cosmétique selon la revendication 1, dans laquelle le dérivé de glycérol possède un point de fusion de -15 à 60°C.

3. Emulsion cosmétique selon la revendication 1 ou 2, dans laquelle le phospholipide est choisi parmi les phosphoglycérides, les lysophosphoglycérides, les sphingomyélines, et leurs mélanges.

4. Emulsion cosmétique selon la revendication 3, dans laquelle le phosphoglycéride est choisi parmi la phosphatidylcholine, la phosphatidyléthanolamine, la phosphatidylsérine, le phosphatidylinositol, le diphosphatidylglycérol, et leurs mélanges.

5. Emulsion cosmétique selon la revendication 3, dans laquelle le phosphoglycéride est une lécithine.

6. Emulsion cosmétique selon la revendication 3, dans laquelle le lysophosphoglycéride est choisi parmi la lysophosphatidylcholine, le lysophosphatidyléthanolamide, la lysophosphatidylsérine, le lysophosphatidylinositol, et leurs mélanges.

7. Emulsion cosmétique selon l'une quelconque des revendications précédentes, dans laquelle le dérivé de glycérol est choisi parmi les monoalcanoates de glycéryle dans lesquels le groupe alcanoate comporte de 2 à 20 atomes de carbone, les monoalcénoates de glycéryle dans lesquels le groupe alcénoate comprend de 3 à 20 atomes de carbone, et leurs mélanges.

8. Emulsion cosmétique selon l'une quelconque des revendications 1 à 6, dans laquelle le dérivé de glycérol est un diglycéride choisi parmi les dialcanoates de glycéryle dans lesquels les groupes alcanoates comportent chacun de 2 à 20 atomes de carbone, les dialcénoates de glycéryle dans lesquels les groupes alcénoates comportent chacun de 3 à 20 atomes de carbone, et leurs mélanges.

9. Emulsion cosmétique selon l'une quelconque des revendications précédentes, sous forme d'un rouge à lèvres à base d'émulsion eau dans huile.

10. Emulsion cosmétique selon la revendication 9, qui comprend en outre, une quantité suffisante de pigment.

11. Emulsion cosmétique selon l'une quelconque des revendications précédentes, dans laquelle moins de 65% en poids des constituants de l'émulsion cosmétique sont liquides à la température ambiante.
